# EUROPEAN PATENT SPECIFICATION

(11) **EP 3 202 447 B1**
(45) Date of publication and mention of the grant of the patent: **12.03.2025**
(21) Application number: 15847661.4
(22) Date of filing: 20.08.2015
(51) Int. Cl.: A61M 5/32, A61M 5/158, A61M 5/315

(54) **SYRINGE ASSEMBLY, PRE-FILLED SYRINGE, SEAL CAP FOR SHEATH WITH PUNCTURE NEEDLE, AND SYRINGE ASSEMBLY PACKAGE**
SPRITZENANORDNUNG, VORGEFÜLLTE SPRITZE, DICHTKAPPE FÜR HÜLLE MIT PUNKTIONSNADEL UND SPRITZENANORDNUNGSVERPACKUNG
ENSEMBLE DE SERINGUE, SERINGUE PRÉREMPLIE, CAPUCHON D'ÉTANCHÉITÉ POUR GAINE AVEC AIGUILLE DE PONCTION, ET EMBALLAGE D'ENSEMBLE DE SERINGUE

(30) Priority: 02.10.2014 JP 2014203838
(43) Date of publication of application: 09.08.2017
(73) Proprietor: Terumo Kabushiki Kaisha, Tokyo 151-0072 (JP)
(72) Inventor: ABE, Yoshihiko, Kanagawa 259-0151 (JP); YOSHIMOTO, Tsuyoshi, Fujinomiya-shi Shizuoka 418-0004 (JP); FUKUSHI, Keiko, Kanagawa 259-0151 (JP)
(74) Representative: Casalonga
(86) International application number: PCT/JP2015/073417
(87) International publication number: WO 2016/051989

(56) References cited:
- EP-A1- 0 027 028
- EP-A1- 2 548 597
- JP-A- 2006 314 554
- JP-A- 2011 014 917
- JP-A- 2011 021 048
- JP-A- 2012 071 046
- JP-A- 2014 036 876
- JP-A- H05 316 595
- US-A1- 2011 027 149

## Description

### TECHNICAL FIELD

The present invention relates to a syringe assembly in which the seal cap is mounted on an outer cylinder, a prefilled syringe, a seal cap for a piercing needle-attached outer cylinder, and a syringe assembly packaging body.

In particular, the present invention relates to a syringe assembly according to the preamble of claim 1 and a seal cap according to the preamble of claim 10, such as they are for example known from EP 2 548 597 A1.

### BACKGROUND ART

As an insulin syringe and the like for administering a small amount of a medical agent to a patient, a syringe having a piercing needle fixed to the distal end of an outer cylinder is used. In constructing a prefilled syringe in which the medical agent is filled in advance by using a syringe of this type, it is necessary to seal the tip of the syringe. Caps capable of sealing the tip of the syringe are proposed, as disclosed in patent document 1 (Japanese translation of PCT International Application Publication No. 2010-534546) and patent document 2 (USP6719732).

### PRIOR ART DOCUMENTS

### PATENT DOCUMENTS

Patent document 1: Japanese translation of PCT International Application Publication No. 2010-534546
Patent document 2: USP6719732
Patent document 3: Japanese Patent Application Laid-Open Publication No. 2013-43957

### SUMMARY OF THE INVENTION

### PROBLEMS TO BE SOLVED BY THE INVENTION

The seal cap (shield 10) of the patent document 1 covers the tip of the injection device (a part of the injection device is shown in Fig. 2 of patent document 1). The tip of the injection device 3 has the hub 2 to which the needle 6 is fixed. The shield 10 has the open proximal end 11, the closed tip 12, and the wall 13 extended from the proximal end 11 to the closed tip 12. The inner surface14 of the wall 13 defines the cavity 15 accommodating a part of the tip of the syringe 3. For example, before using an injection device in a case where the shield 10 is fixed to the tip of the syringe to protect the tip while the injection device is being transported, the portion 14a of the inner surface 14 contacts the hub 2 disposed at the tip of the syringe 3.

In the seal cap (device for protecting the syringe needle) of the patent document 2, as shown in Figs. 1 through 5 of the patent document 2, the elastic needle cap 20 extended longitudinally between the open proximal end 22 and the closed tip 24 has the inner housing 26 partitioned by the lateral direction wall 28 and the end wall 30. Between the first and second portions 40 and the second portion 42 of the housing 26, the annular bead (rib) 70 expanded inward is formed at the edge of the second portion 42 extended toward the proximal end 22. To improve the deformability of the annular bead 70 and facilitate the passing of the pressurized water vapor through the annular bead 70, four slots 72 longitudinally extended are formed on the bead 70.

As is often the case, the needle-attached prefillable syringe of this type is provided as the prefilled syringe in which a liquid medicine is filled. Thus, the seal cap having the function of sealing the needle tip of the outer cylinder is mounted on the outer cylinder. It is often the case that to seal the needle tip, the seal cap is formed by molding an elastic rubber material into which the needle tip can be pierced. In the production process of the prefilled syringe, a static electricity removal apparatus (ionizer) is installed as a measure against the sticking of foreign matters to products. It is known that the ionizer generates ozone. In dependence on the quality of a material for the seal cap, the seal cap may have a low resistance to ozone. Thus, there is a fear that the seal cap may deteriorate in the above-described production process. 20 to 40 ppb of ozone is present in a normal environment. Thus, there is a possibility that the seal cap is deteriorated by the ozone. The present inventors have found that in a case where the seal cap is stored in the presence of the ozone, the seal cap deteriorates and cracks and at its stress-applied portion, for example, a portion where the seal cap is fitted on the outer cylinder. The present inventors examined the addition of an additive to rubber to prevent the seal cap from deteriorating. There is no denying the possibility that a medical agent filled inside the prefilled syringe may denature owing to the addition of the additive to the rubber. Therefore, it is desirable to impart ozone resistance to the seal cap by avoiding the use of the additive for the rubber as much as possible.

The needle cap, disclosed in a patent document 3, for protecting the needle mounted on the distal end of the syringe barrel is formed of the rubber composition containing 70 to 100% by mass of the ethylene-propylene-diene rubber in the base polymer thereof. The needle cap disclosed in the patent document 3 is excellent in that it is not deteriorated by ozone. But it occurred that the material for forming the seal cap sticks to the outer surface of the needle tip of the piercing needle, as described later.

The syringe assembly of the above-described type is provided in a state where the seal cap has been mounted on the outer cylinder with the piercing needle being pierced into the seal cap. Therefore, the state in which the piercing needle is pierced into the seal cap, in other words, the state in which the outer surface of the distal portion of the piercing needle is in close contact with the material forming the seal cap continues for a certain period of time from the time of the production of the syringe assembly including sterilization process, transport, and storage till the use thereof. To decrease the piercing resistance at a piercing time, normally, silicone is applied to the piercing needle. Nevertheless, in dependence on a storage environment, when the seal cap is removed from the outer cylinder, it occurs that the material forming the seal cap sticks to the outer surface of the piercing needle. The sticking of the material forming the seal cap to the piercing needle may cause an increase of the piercing resistance, which is undesirable.

Therefore, it is an object of the present invention to provide a syringe assembly which is allowed to have ozone resistance by avoiding the use of an additive as much as possible for a material forming a seal cap and in which the material forming the seal cap hardly sticks to a piercing needle with the lapse of a certain period of time, a prefilled syringe, a seal cap for a piercing needle-attached outer cylinder, and a syringe assembly packaging body.

### MEANS FOR SOLVING THE PROBLEMS

The above-described object can be achieved by a syringe assembly having the following form:
The syringe assembly is composed of an outer cylinder having an outer cylinder body part, a piercing needle mounting part provided at a distal end portion of the outer cylinder body part, a piercing needle which has a piercing needle tip at a distal end thereof and whose proximal end portion is fixed to the piercing needle mounting part and a seal cap mounted on the outer cylinder.

The seal cap is composed of a closed distal end part, an open proximal end part, a hollow part including a piercing needle mounting part-accommodating part positioned at a distal side from the open proximal end part and accommodating a distal end portion of the piercing needle mounting part and a piercing needle accommodating part continuous with a distal end of the piercing needle mounting part-accommodating part and accommodating the piercing needle, and a pierceable part into which the piercing needle tip of the piercing needle accommodated inside the piercing needle accommodating part is pierceable.

The seal cap is formed of a rubber composition containing a mixture of butyl rubber and diene-based rubber as a base polymer thereof and is mounted on the outer cylinder with the piercing needle tip being pierced into the pierceable part of the seal cap.

The above-described object can be achieved by a prefilled syringe having the following form:
The prefilled syringe is composed of a syringe assembly, a gasket which is accommodated inside the outer cylinder and liquid-tightly slidable inside the outer cylinder, and a medical agent filled inside a space formed of the outer cylinder and the gasket.

The above-described object can be achieved by the seal cap for the outer cylinder having the following form:
The seal cap for the piercing needle-attached outer cylinder is mounted on the outer cylinder having an outer cylinder body part, a piercing needle mounting part provided at a distal end portion of the outer cylinder body part, and a piercing needle which has a piercing needle tip at a distal end thereof and whose proximal end portion is fixed to the piercing needle mounting part.

The seal cap is composed of a closed distal end part, an open proximal end part, a hollow part including a piercing needle mounting part-accommodating part positioned at a distal side from the open proximal end part and accommodating the piercing needle mounting part and a piercing needle accommodating part continuous with a distal end of the piercing needle mounting part-accommodating part and accommodating the piercing needle, a pierceable part into which the piercing needle tip of the piercing needle accommodated inside the piercing needle accommodating part is pierceable, and a projected part formed on an inner surface of the piercing needle mounting part-accommodating part.

The seal cap is formed of a rubber composition containing the mixture of butyl rubber and diene-based rubber as the base polymer thereof.

The above-described object can be achieved by a packaging body accommodating a plurality of syringe assemblies.

The packaging body is composed of a container body whose upper surface is open and which has shape retainability, an outer cylinder holding member capable of holding a plurality of the syringe assemblies, a plurality of the syringe assemblies held by the outer cylinder holding member, and a sheet-shaped lid member which airtightly seals the open upper surface of the container body and is peelable therefrom, and an air-permeable part, having bacteria impermeability and sterilizing gas permeability, which is provided on the container body or on the sheet-shaped lid member, , and said packaging body has subjected to high-pressure steam sterilization or ethylene oxide gas sterilization.

The invention is defined in claim 1. Advantageous embodiments are set out in the dependent claims.

### BRIEF DESCRIPTION OF THE DRAWINGS

Fig. 1 is a front view of a prefilled syringe of an embodiment of the present invention.
Fig. 2 is a sectional view taken along a line A-A of Fig. 1.
Fig. 3 is an enlarged sectional view of a syringe assembly of the present invention for use in the prefilled syringe shown in Figs. 1 and 2.
Fig. 4 is an enlarged front view of a seal cap for an outer cylinder for use in the prefilled syringe shown in Figs. 1 and 2 and the syringe assembly shown in Fig. 3.
Fig. 5 is a bottom view of the seal cap for the outer cylinder shown in Fig. 4.
Fig. 6 is an enlarged sectional view taken along a line B-B of Fig. 4.
Fig. 7 is a front view of the outer cylinder for use in the prefilled syringe shown in Figs. 1 and 2 and the syringe assembly shown in Fig. 3.
Fig. 8 is a sectional view taken along a line C-C of Fig. 7.
Fig. 9 is a perspective view of the outer cylinder shown in Fig. 7.
Fig. 10 is an explanatory view for explaining the action of the seal cap for use in the outer cylinder of the present invention.

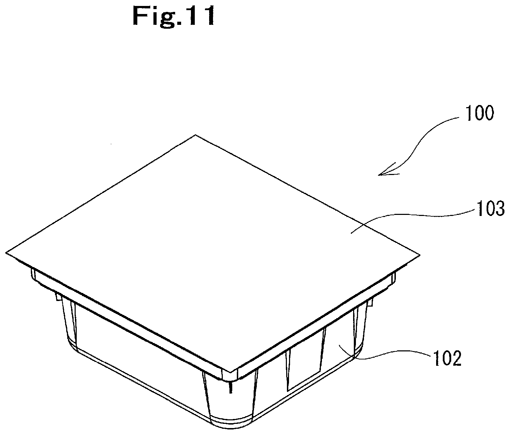

Fig. 11 is a perspective view of a syringe assembly packaging body of the present invention.

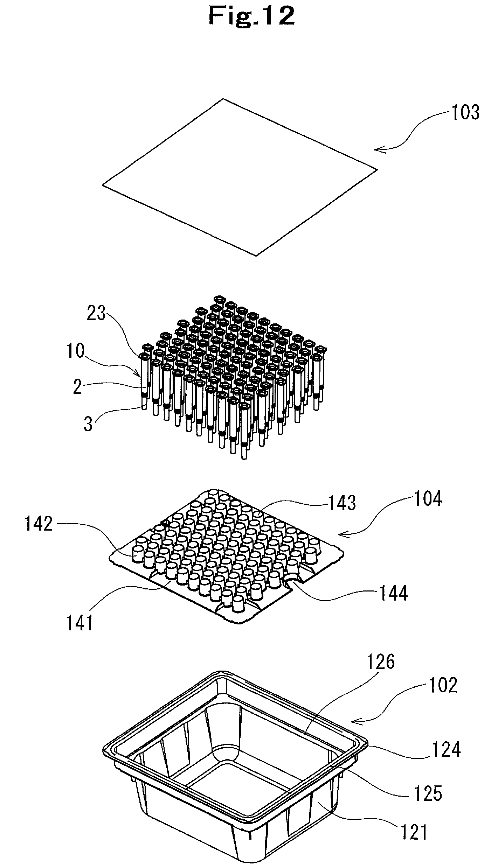

Fig. 12 is an explanatory view for explaining an inner form of the syringe assembly packaging body shown in Fig. 11.

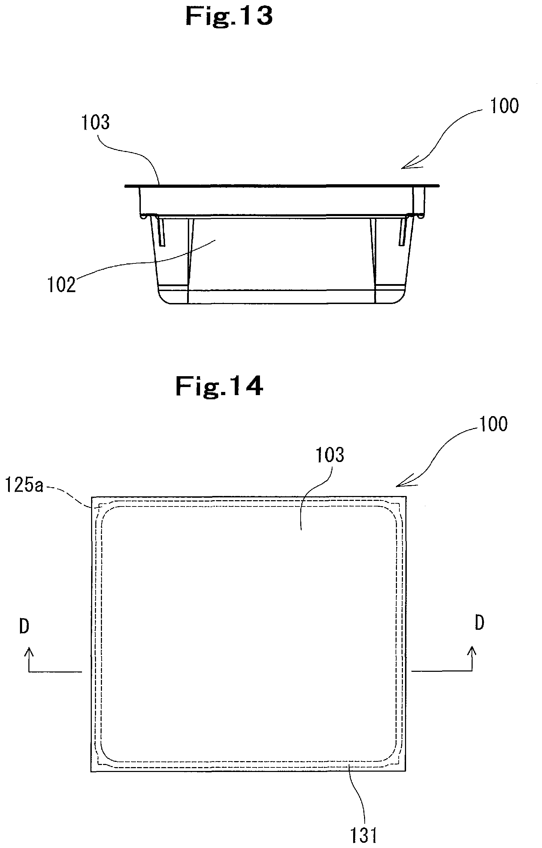

Fig. 13 is a front view of the syringe assembly packaging body shown in Fig. 11.
Fig. 14 is a plan view of the syringe assembly packaging body shown in Fig. 13.

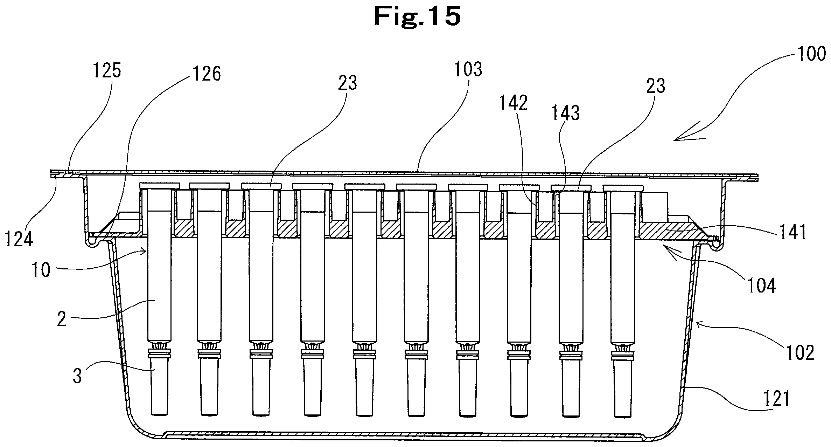

Fig. 15 is an enlarged sectional view taken along a line D-D of Fig. 14.

### MODE FOR CARRYING OUT THE INVENTION

By using the embodiments shown in the drawings, description is made below on a seal cap of the present invention for a piercing needle-attached outer cylinder, a syringe assembly in which the seal cap is mounted on the piercing needle-attached outer cylinder, and a prefilled syringe using the syringe assembly in which the seal cap is mounted on the piercing needle-attached outer cylinder.

A prefilled syringe 1 of the present invention is composed of a syringe assembly 10, a gasket 4 which is accommodated inside the syringe assembly and liquid-tightly slidable inside the syringe assembly 10, and a medical agent 8 filled inside a space formed of the syringe assembly 10 and the gasket 4.

The syringe assembly (in other words, piercing needle-equipped outer cylinder mounted a cap) 10 of the present invention is composed of an outer cylinder 2 having an outer cylinder body part 21 having a piercing needle fixing part 22 at its distal end and a piercing needle 6 which has a piercing needle tip 61 at its distal end and whose proximal end portion is fixed to the piercing needle fixing part 22; and a seal cap 3 mounted on the outer cylinder 2.

The seal cap 3 has a closed distal end part 31, an open proximal end part 32, a cylindrical hollow part 30 extended from the open proximal end part 32 toward a distal end of the seal cap and being capable of accommodating the piercing needle fixing part 22 therein, and a pierceable part 33 into which the piercing needle tip 61 of the piercing needle 6 inserted into the hollow part 30 is pierceable. The seal cap 3 is formed of a rubber composition containing a mixture of butyl rubber and diene-based rubber as its base polymer. The seal cap 3 is mounted on the outer cylinder 2 with the piercing needle tip 61 of the piercing needle 6 being pierced into the pierceable part 33.

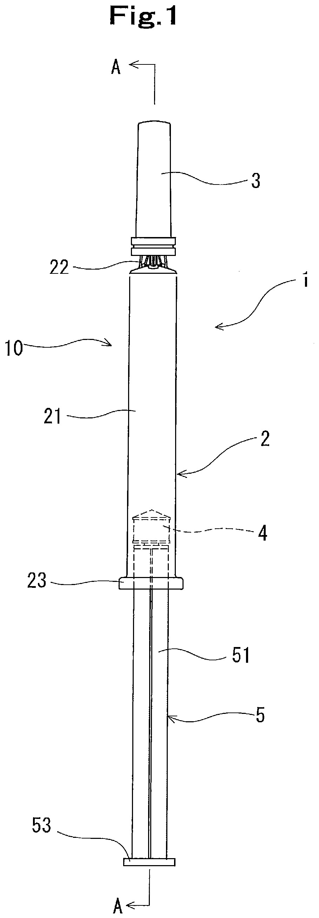

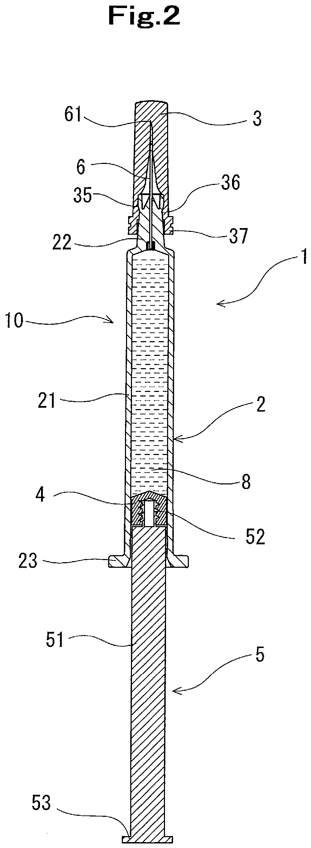

As shown in Figs. 1 and 2, the prefilled syringe 1 is composed of the syringe assembly 10, which consists of the outer cylinder 2 and the seal cap 3 mounted on the outer cylinder 2 so as to seal the needle tip of the piercing needle, the gasket 4 which is accommodated inside the syringe assembly 10 and liquid-tightly slidable inside the syringe assembly 10, the medical agent 8 filled inside the space formed of the syringe assembly 10 and the gasket 4, and a plunger 5 mounted on the gasket 4 in advance or when the prefilled syringe is used.

The medical agent 8 is filled inside the space formed of the outer cylinder 2, the gasket 4, and the seal cap 3.

It is possible to use any medical agent to be used as injection solutions as the medical agent 8 to be filled inside the above-described space. Examples of the medical agent 8 include protein pharmaceuticals such as an antibody, peptide pharmaceuticals such as hormone, nucleic acid medicines, cell medicines, blood products, vaccines for protecting infectious diseases, anticancer drugs, anesthetics, jolt, antibiotics, steroid drugs, proteolytic enzyme inhibitor, heparin, sugar injections such as glucose, an electrolyte correction injection solution such as sodium chloride, potassium lactate, and the like, vitamin preparations, fat emulsion, an imaging agent, and a stimulant drug.

The outer cylinder 2 has the outer cylinder body part 21, the cylindrical (hollow) piercing needle fixing part 22 provided at the distal end portion of the outer cylinder body part 21, a flange 23 provided at a proximal end portion of the outer cylinder body part 21, and the piercing needle 6 whose proximal end portion is fixed to the piercing needle fixing part 22.

The piercing needle 6 has the piercing needle tip 61 at its distal end. The proximal end portion of the piercing needle 6 is inserted into a hollow portion of the piercing needle fixing part 22 and fixed thereto. The inside of the piercing needle 6 communicates with an inner space 20 of the outer cylinder 2. In other words, the piercing needle 6 is embedded in the piercing needle fixing part 22 of the outer cylinder 2 at a portion disposed a little distally from its proximal end (proximal end portion not including proximal end). Thus, the hollow portion of the piercing needle fixing part 22 has disappeared. As the piercing needle 6, a metallic hollow needle having its needle tip is used. In this embodiment, a lubricant is applied to at least the piercing needle tip 61 of the piercing needle 6. Although the lubricant is not limited to a specific one, silicone oil is used in this embodiment. By using the silicone oil, it is possible to expect a decrease in piercing resistance at a piercing time.

The piercing needle 6 may be inserted into the hollow portion of the piercing needle fixing part 22 of the outer cylinder 2 molded in advance and fixed to the piercing needle fixing part 22 by means of an adhesive agent, thermal welding or the like. The piercing needle 6 may be fixed to the outer cylinder 2 by insert molding. In the case of the insert molding, by forming the outer cylinder 2 by molding a material, the piercing needle fixing part 22 has a cylindrical (hollow) configuration into which the piercing needle 6 has been inserted with the proximal end portion of the piercing needle 6 being inserted into the hollow portion of the piercing needle fixing part 22 and fixed thereto.

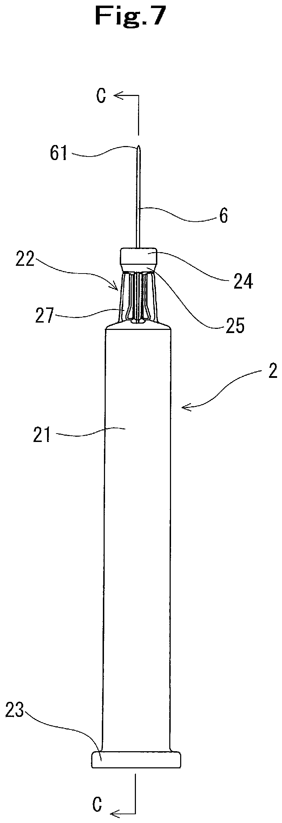

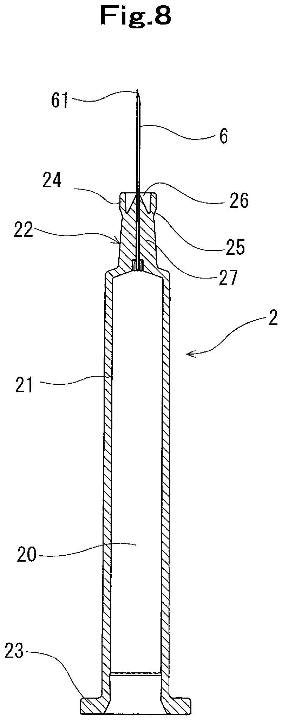

The outer cylinder 2 is transparent or semitransparent. The outer cylinder body part 21 is a substantially cylindrical portion which accommodates the gasket 4 liquid-tightly and slidably. The piercing needle fixing part 22 is a hollow cylindrical part projecting forward from the distal end portion (shoulder portion) of the outer cylinder body and having a smaller diameter than the outer cylinder body. As shown in Figs. 7 and 8, the piercing needle fixing part 22 has a head portion 24 provided at its distal end, a short-tapered diameter-reduced portion 25 which is provided at the proximal end of the head portion 24 and becomes shorter toward its proximal end in its diameter, and a connecting portion 27 connecting a proximal end portion of the tapered diameter-reduced portion 25 and the distal end portion of the outer cylinder body part 21 to each other. A concave portion is formed of the tapered diameter-reduced portion 25. In the head portion 24, there are formed a concavity 26 recessed from a distal end surface of the head portion 24 toward its proximal end and a hollow conical portion which is positioned inside the concavity 26 and has an apex at its distal end side.

A plurality of grooves extended in the axial direction of the outer cylinder 2 is formed on an outer surface of the connecting portion 27. The concave portion 25 may not be tapered, but may have a diameter-decreased configuration so that the concave portion is stepped from the proximal end of the head portion 24. In addition, it is possible to omit the formation of the connecting portion 27 and directly connect the proximal end portion of the concave portion (tapered diameter-reduced portion 25) and the distal end portion of the outer cylinder body part 21 to each other. It is also possible to omit the formation of the concavity 26 and the conical portion so as to shape the head portion 24 hollowly and columnarly (cylindrically). It is preferable to form the concave portion 25 annularly as in the case of this embodiment.

Examples of a material for forming the outer cylinder 2 include resins such as polypropylene, polyethylene, polystyrene, polyamide, polycarbonate, polyvinyl chloride, poly-(4-methylpentene-1), acrylic resin, acrylonitrile-butadiene-styrene copolymer, polyester including polyethylene terephthalate, cyclic olefin polymers, and cyclic olefin copolymers. Of these resins, the resins such as the polypropylene, the cyclic olefin polymers, and the cyclic olefin copolymers are preferable because these resins can be easily molded and are heat-resistant.

As the piercing needle 6, a hollow one having the piercing needle tip 61 at its distal end is used. As a material for forming the piercing needle 6, metals are normally used. Of metals, stainless steel is preferable.

As shown in Figs. 1 and 2, the gasket 4 has a body part extended in substantially the same diameter and a plurality of annular ribs (Two annular ribs are formed in this embodiment. When two or more annular ribs are formed, the number thereof is not limited, provided that liquid tightness and slidability are satisfied) formed on the body part. The ribs liquid-tightly contact the inner surface of the outer cylinder 2. A distal end surface of the gasket 4 has a configuration corresponding to that of the inner surface of the distal end of the outer cylinder 2 to prevent the formation of a gap between both surfaces as much as possible when both surfaces contact each other.

As a material for forming the gasket 4, it is preferable to use elastic rubber (for example, isoprene rubber, butyl rubber, latex rubber, silicone rubber, and the like); and synthetic resin (for example, styrene elastomer such as SBS elastomer, SEBS elastomer, and the like and olefin elastomer such as ethylene-α-olefin copolymer elastomer).

The gasket 4 has a concave portion extended inward from its proximal end portion. The concave portion of the gasket 4 is female screw-shaped and engageable with a male screw portion formed on an outer surface of a projected portion 52 formed at a distal end portion of the plunger 5. Engagement between the female and male screw portions prevents the plunger 5 from being removed from the gasket 4. The plunger 5 may be removed from the gasket and mounted thereon when the prefilled syringe is used. The plunger 5 has the projected portion 52 projected forward cylindrically from a disk portion disposed at its distal end. A male screw which threadedly engages the concave portion of the gasket 4 is formed on an outer surface of the projected portion. The plunger 5 has a body part 51 cross-shaped and axially extended and a pressing disk portion 53 provided at its proximal end portion.

The seal cap 3 of the present invention for the piercing needle-attached outer cylinder is used by mounting the seal cap on the outer cylinder having the outer cylinder body part 21, the cylindrical piercing needle fixing part 22 provided at the distal end portion of the outer cylinder body part 21 and having the head portion 24, and the piercing needle 6 which has the piercing needle tip 61 at its distal end and the proximal end portion of which is inserted into the piercing needle mounting part 22 and fixed thereto.

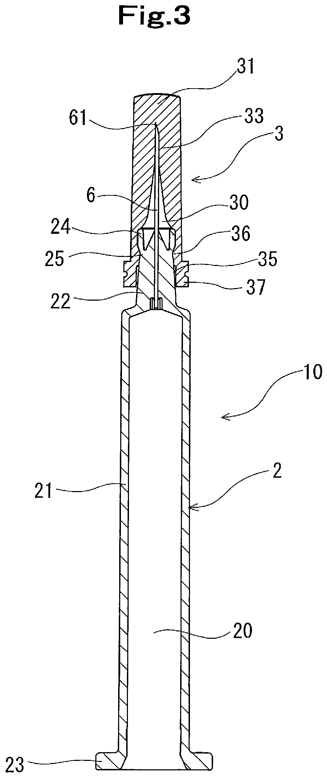

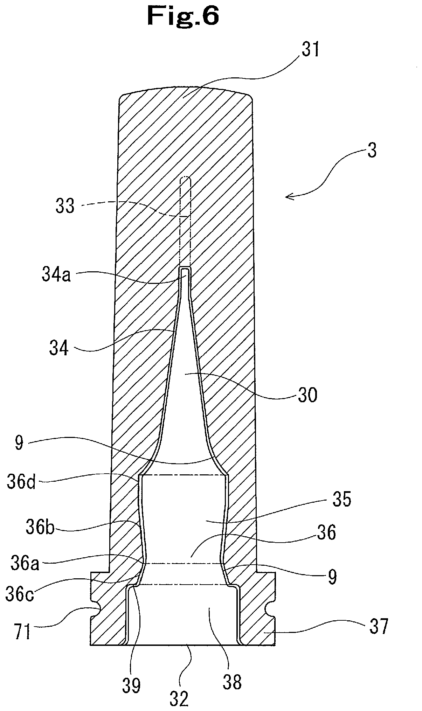

As shown in Fig. 6, the seal cap 3 has the closed distal end part 31, the open proximal end part 32, the hollow part 30 having the piercing needle fixing part-accommodating part 35 positioned distally from the open proximal end part 32 and accommodating the piercing needle fixing part 22 and the tapered piercing needle accommodating part 34 continuous with the distal end of the piercing needle fixing part-accommodating part 35, the pierceable part 33 into which the piercing needle tip 61 of the piercing needle 6 accommodated inside the piercing needle accommodating part 34 can be pierced, and a projected part 36 formed on the inner surface of the piercing needle fixing part-accommodating part 35. As shown in Fig. 3, in the seal cap 3 of this embodiment mounted on the outer cylinder 2, at least a part of the hollow part 30 (more specifically, the piercing needle fixing part-accommodating part 35 which is a part of the hollow part and the vicinity thereof) is expansively spread outward by the piercing needle fixing part 22 accommodated inside the hollow part 30.

The seal cap is formed of the rubber composition containing the mixture of butyl rubber and diene-based rubber as its base polymer. It is preferable to crosslink the butyl rubber and diene-based rubber of the rubber composition. It is possible to crosslink the butyl rubber and the diene-based rubber by adding a crosslinking agent capable of crosslinking them. It is possible to add a catalyst to the butyl rubber and the diene-based rubber as necessary. It is preferable that the diene-based rubber contains butadiene rubber as its main component. The diene-based rubber may consist of 100% butadiene rubber. It is preferable for the diene-based rubber to contain the butadiene rubber at not less than 70% or not less than 30% thereof. As the diene-based rubber, in addition to the above-described butadiene rubber, butadiene-based rubbers such as styrene-butadiene rubber, acrylonitrile-butadiene rubber, acrylonitrile-butadiene-isoprene rubber, butadiene-acrylic acid ester rubber, and hydrogenated styrene-butadiene rubber are preferable.

The ratio between the butyl rubber of the base polymer and the diene-based rubber thereof is set favorably to 7:3 to 4:6 and more favorably to 5:5 to 6:4. The rubber composition may contain a filler in addition to the base polymer. The filler to be used in the present invention is not limited to specific one, but it is possible to appropriately use fillers such as an inorganic filler and an organic filler usually used for the rubber composition. Examples of the inorganic filler include silica, fused silica, talc, alumina, mica, clay, hydrotalcite, aluminum hydroxide, barium sulfate, calcium hydroxide, calcium carbonate, magnesium carbonate, aluminum borate, potassium titanate, calcium silicate, boron nitride, aluminum nitride, aluminum oxide, titanium oxide, zirconium oxide, calcium carbonate, magnesium sulfate, and zinc oxide. As the organic filler, carbon materials such as graphite, carbon black, and carbon fiber, aromatic polyamide fiber, aromatic polyamide-based pulp-like fiber, and glass fiber are used. The above-described fillers can be used singly or in combination of not less than two kinds. The ratio between the base polymer of the rubber composition and the filler thereof is set favorably to 5:5 to 8:2 and more favorably to 6:4 to 7:3.

It is preferable that a material for forming the seal cap of the present invention contains a release agent. The amount of the release agent is set to favorably 0.3 to 5 parts by weight and more favorably 0.3 to 3 parts by weight for 100 parts by weight of the base polymer. As the release agent, it is possible to list high fatty acid amide such as stearic acid amide, oleic acid amide, palmitic acid amide; high fatty acid ester such as ethyl oleate; high fatty acid amine such as stearylamine and oleylamine; ester of aliphatic alcohol; and paraffin. But the release agent to be used in the present invention is not limited to the above-described ones.

The material for forming the seal cap of the present invention may contain a pigment such as carbon black, titanium oxide, and the like as a colorant and paraffin oil and the like as a plasticizer as necessary.

The inner surface of the piercing needle mounting part-accommodating part 35 of the seal cap 3 and the outer surface of the piercing needle mounting part 22 of the outer cylinder 2 are in close contact with each other. In the seal cap 3 shown in Fig. 6, the sticking restraining film 9 for restraining the inner surface of the piercing needle mounting part-accommodating part 35 and the outer surface of the piercing needle mounting part 22 of the outer cylinder 2 from sticking to each other is formed on at least the inner surface of the piercing needle mounting part-accommodating part 35. The sticking restraining film 9 may be formed not entirely on the inner surface of the piercing needle mounting part-accommodating part 35, but at only a portion of the inner surface thereof which closely contacts the outer surface of the piercing needle mounting part 22.

In the seal cap 3 of this embodiment, the sticking restraining film 9 is formed on the entire inner surface of the piercing needle accommodating part 34. Therefore, when the piercing needle tip 61 of the piercing needle 6 contacts the inner surface of the piercing needle accommodating part 34, the piercing needle does not stick to the inner surface thereof. Thereby in mounting the seal cap 3 on the piercing needle mounting part 22 of the outer cylinder 2, the piercing needle tip 61 of the piercing needle 6 which has entered the piercing needle accommodating part 34 is guided to the distal end (small-diameter distal end portion 34a) of the piercing needle accommodating part 34 without sticking to the inner surface of the piercing needle accommodating part 34 and is securely pierced into the pierceable part 33.

In the seal cap 3 of this embodiment, the sticking restraining film 9 is formed entirely on the inner surface of the piercing needle mounting part-introducing portion 38. Thus, when the piercing needle mounting part 22 enters the piercing needle mounting part-introducing portion 38, it does not occur that the outer surface of the piercing needle mounting part 22 sticks to the inner surface of the piercing needle mounting part-introducing portion 38. Thereby it is possible to smoothly mount the seal cap 3 on the piercing needle mounting part 22 of the outer cylinder 2.

As shown in Fig. 3, the syringe assembly 10 of the present invention has a state in which the seal cap 3 is mounted on the distal end portion (piercing needle mounting part 22) of the outer cylinder 2, the piercing needle tip 61 of the piercing needle 6 is pierced into the pierceable part 33 of the seal cap 3 and is sealed liquid-tightly, the concave portion 25 positioned proximally from the head portion 24 of the piercing needle mounting part 22 and the projected part 36 formed on the inner surface of the piercing needle mounting part-accommodating part 35 engage each other, and the inner surface of the piercing needle mounting part-accommodating part 35 and the outer surface of the piercing needle mounting part 22 are in close contact with each other. As shown in Fig. 3, in the seal cap 3 of this embodiment mounted on the outer cylinder 2, at least a part of the hollow part 30 (more specifically, the piercing needle fixing part-accommodating part 35 which is a part of the hollow part and the vicinity thereof) is expansively spread outward by the piercing needle fixing part 22 accommodated inside the hollow part 30.

In the syringe assembly 10, the inner surface of the piercing needle mounting part-accommodating part 35 and the outer surface of the piercing needle mounting part 22 can be brought into close contact with each other through the sticking restraining film 9. By bringing the inner surface of the piercing needle mounting part-accommodating part 35 and the outer surface of the piercing needle mounting part 22 into close contact with each other through the sticking restraining film 9, it is possible to restrain the inner surface of the piercing needle mounting part-accommodating part 35 and the outer surface of the piercing needle mounting part 22 from sticking to each other in a case where the syringe assembly is stored with the seal cap 3 being mounted on the outer cylinder 2 and even in a case where the syringe assembly undergoes high-pressure steam sterilization or ethylene oxide gas sterilization which subjects the syringe assembly to a pressure load-applied state. Similarly, in the prefilled syringe 1 of the present invention, the inner surface of the piercing needle mounting part-accommodating part 35 and the outer surface of the piercing needle mounting part 22 are kept in close contact with each other through the sticking restraining film 9. Thus, in a case where the syringe assembly is stored with the seal cap 3 being mounted on the outer cylinder 2 and even in a case where the syringe assembly undergoes the high-pressure steam sterilization or the ethylene oxide gas sterilization which subjects the syringe assembly to the pressure load-applied state, it is possible to restrain the inner surface of the piercing needle mounting part-accommodating part 35 and the outer surface of the piercing needle mounting part 22 from sticking to each other.

Although in the above-described embodiment, the sticking restraining film 9 is formed on the inner surface of the piercing needle mounting part-accommodating part 35, the portion where the sticking restraining film is formed is not limited thereto, but may be formed on the outer surface of the piercing needle mounting part 22 of the outer cylinder 2. Further, the sticking restraining film may be formed on both the inner surface of the piercing needle mounting part-accommodating part 35 of the seal cap 3 and on the outer surface of the piercing needle mounting part 22 of the outer cylinder 2.

It is preferable to subject the syringe assembly to the high-pressure steam sterilization or the ethylene oxide gas sterilization. Because the seal cap 3 to be used in the present invention is gas-permeable, it is possible to subject the entire syringe assembly composed of the outer syringe and the seal cap into which the needle tip of the piercing needle has been pierced to the high-pressure steam sterilization or the ethylene oxide gas sterilization. It is especially preferable to subject the syringe assembly to the high-pressure steam sterilization.

As materials for forming the sticking restraining film 9, it is possible to use fluorine-based resin such as polytetrafluoroethylene (PTFE), tetrafluoroethylene·perfluoroalkyl vinyl ether copolymer resin (PFA), tetrafluoroethylene-hexafluoropropylene copolymer (FEP), polychlorotrifluoroethylene (PCTFE), polyvinylidene fluoride (PVDF); silicon-based resin such as a silicone polymerized film formed of a liquid coating agent containing reactive silicone oil as its main component, polyparaxylylene, diamond-like carbon, and the like.

As a sticking restraining polymerized film, it is preferable to use the silicone polymerized film formed of the liquid coating agent containing the reactive silicone oil as its main component. Because the liquid coating agent containing the reactive silicone oil as its main component is in a liquid form, the sticking restraining film can be easily and securely applied to the inner surface of the seal cap 3 (piercing needle mounting part-accommodating part 35). In addition, the liquid coating agent containing the reactive silicone oil as its main component is polymerized (including crosslinking) at a normal temperature or by heating to form the silicone polymerized film. Thus, the liquid coating agent containing the reactive silicone oil as its main component can be easily and securely formed on the inner surface of the seal cap3 (piercing needle mounting part-accommodating part 35) as the sticking restraining polymerized film. Furthermore, the silicone polymerized film formed of the liquid coating agent containing the reactive silicone oil as its main component is flexible. Thus, when the inner surface of the seal cap 3 (piercing needle mounting part-accommodating part 35) is expansively spread outward by the head portion 24 of the piercing needle fixing part 22 of the outer cylinder 2 in mounting the seal cap 3 on the piercing needle fixing part 22, the head portion 24 of the piercing needle fixing part 22 follows the deformation of the inner surface of the seal cap 3 and thus hardly peels off the inner surface of the seal cap 3 (piercing needle mounting part-accommodating part 35). In this embodiment, the inside of the head portion 24 is hollow, thus forming the annular head portion.

As the reactive silicone oil, it is possible to list polydimethylsiloxanes having functional groups such as a silanol group, an amino group, an epoxy group, an alicyclic epoxy group, a carbinol group, a methacrylic group, a polyether group, a mercapto group, a carboxylic group or a phenol group at both ends thereof. Of these polydimethylsiloxanes, the polydimethylsiloxane having the silanol group at its both ends is preferable. In a case where the polydimethylsiloxane having the silanol group at its both ends is used, the condensate of the reactive silicone oil has a chemically stable siloxane bond in its entire main chain. Thus, the condensate of the reactive silicone oil is capable of forming the silicone polymerized film which hardly cause interaction (for example, adsorption of medical agent) with the medical agent 8 filled inside the outer cylinder 2.

As shown in Fig. 3, the syringe assembly 10 of the present invention has the state in which the seal cap 3 is mounted on the distal end portion (piercing needle fixing part 22) of the outer cylinder 2; the piercing needle tip 61 of the piercing needle 6 is pierced into the pierceable part 33 of the seal cap 3 and is sealed liquid-tightly, the concave portion 25 (proximal end portion of head portion 24) of the piercing needle fixing part 22 and the projected part 36 formed on the inner surface of the piercing needle fixing part-accommodating part 35 engage each other, and the inner surface of the piercing needle fixing part-accommodating part 35 and the outer surface of the piercing needle fixing part 22 are in close contact with each other through the sticking restraining film 9. At this time, at least the piercing needle fixing part-accommodating part 35 is expansively spread outward by the piercing needle fixing part 22.

The form of the inner surface of the seal cap 3 is described below.

The seal cap 3 has the projected part 36, formed on the inner surface thereof, which is located at a position distal from the open proximal end part 32 at a predetermined length. The projected part 36 has an apex 36a projected to a highest extent and an inclined portion (tapered portion) 36b which is extended from the apex 36a toward the distal end of the seal cap and becomes gradually lower in its projection height toward the distal end of the seal cap. In this embodiment, the projected part 36 is formed as an annular projected part. The inclined portion 36b is formed as a tapered portion in which the inner diameter of the piercing needle fixing part-accommodating part 35 decreases toward the proximal end of the seal cap.

At the apex 36a, the inner diameter of the piercing needle fixing part-accommodating part 35 is set a little smaller than the outer diameter of the head portion 24 of the piercing needle fixing part 22 of the outer cylinder 2. Thereby when the seal cap 3 is mounted on the piercing needle fixing part 22 of the outer cylinder 2, the piercing needle fixing part-accommodating part 35 which forms a part of the hollow part and its vicinity are placed in a state in which the piercing needle fixing part-accommodating part 35 and its vicinity are expansively spread outward by the piercing needle fixing part 22 accommodated inside the hollow part 30 as shown in Fig. 3. Thereby the inner surface of the piercing needle fixing part-accommodating part 35 of the seal cap 3 is pressed against the outer surface of the head portion 24 through the sticking restraining film 9 and closely contacts the outer surface of the head portion 24. Further, in this embodiment, the projected part 36 of the seal cap 3 and the concave portion 25 are in engagement with each other. In a state where the seal cap 3 is mounted on the piercing needle fixing part 22, the distal end side inclined portion 36b is extended toward the distal end of the seal cap beyond the concave portion 25. The inner diameter of the piercing needle fixing part-accommodating part 35 in the vicinity of at least the distal end portion of the distal end side inclined portion 36b is set a little smaller than the outer diameter of the head portion 24 of the piercing needle fixing part 22 of the outer cylinder 2.

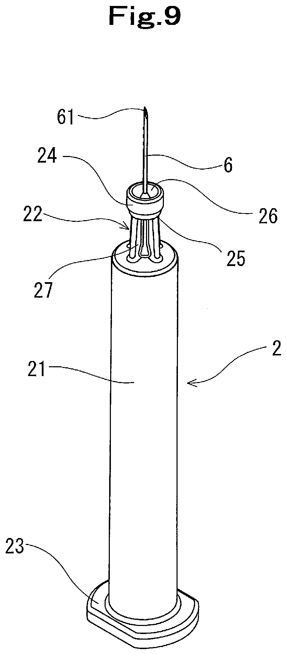

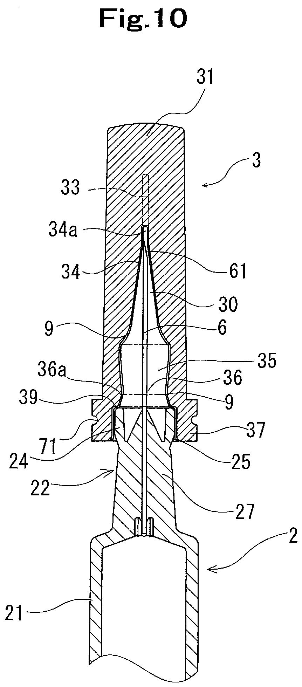

Thereby as shown in Figs. 9 and 10, when the seal cap 3 is mounted on the piercing needle fixing part 22 of the outer cylinder 2, the inner surface of the distal end side inclined portion 36b is entirely pressed against the outer surface of the head portion 24 through the sticking restraining film 9 and closely contacts the outer surface of the head portion. Thereby it is possible to reduce undesired removal of the seal cap 3 from the outer cylinder 2 to a higher extent. Further, because the distal end side inclined portion 36b is pressed against the outer surface of the head portion 24 through the sticking restraining film 9, at least the piercing needle fixing part-accommodating part 35 is placed in a state in which it is expansively spread outward by the heat portion 24.

In the seal cap 3 of this embodiment, as shown in Fig. 6, the projected part 36 further includes a proximal end side inclined portion 36c which extends from the apex 36a toward an open end (proximal end) of the seal cap and gradually decreases in its projection height toward the open end (proximal end) of the seal cap. Thereby in mounting the seal cap 3 on the piercing needle fixing part 22 of the outer cylinder 2, the apex 36a of the projected part 36 is capable of easily climbing over the head portion 24 of the piercing needle fixing part 22 from the distal end side of the head portion 24.

In the seal cap 3 of this embodiment, the piercing needle fixing part-accommodating part 35 has a linear portion 36d extended at a predetermined length (more specifically, extended to the proximal end portion of the piercing needle accommodating part 34) from a distal end portion of the distal end side inclined portion 36b of the projected part 36 toward the distal end of the seal cap. At the linear portion 36d, the inner diameter of the piercing needle fixing part-accommodating part 35 is constant and a little smaller than the outer diameter of the head portion 24 of the piercing needle fixing part 22 of the outer cylinder 2. Thereby when the seal cap 3 is mounted on the piercing needle fixing part 22 of the outer cylinder 2, the linear portion 36d is pressed against the outer surface of the head portion 24 through the sticking restraining film 9 and consequently closely contacts the outer surface thereof. Because the linear portion 36d is pressed against the outer surface of the head portion 24 through the sticking restraining film 9, at least the piercing needle fixing part-accommodating part 35 is placed in the state in which it is expansively spread outward by the head portion 24.

In this embodiment, the proximal end portion of the distal end side inclined portion 36b of the projected part 36 of the seal cap 3 is positioned on the periphery of the concave portion 25 of the piercing needle fixing part 22 of the outer cylinder 2. The inner diameter of the piercing needle fixing part-accommodating part 35 in the vicinity of at least the proximal end portion of the distal end side inclined portion 36b is set a little smaller than the outer diameter of the annular concave portion 25. Therefore, the distal end side inclined portion 36b of the projected part 36 of the seal cap 3 is pressed against the outer surface of the concave portion 25 through the sticking restraining film 9 and consequently closely contacts the outer surface thereof. Thereby it is possible to reduce undesired removal of the seal cap 3 from the outer cylinder 2 to a higher extent.

In this embodiment, the concave portion 25 consists of the tapered diameter-reduced portion which is provided at the proximal end of the head portion 24 and decreases toward the proximal end of the outer cylinder in its diameter. Thereby in removing the seal cap 3 from the outer cylinder 2, the projected part 36 of the seal cap 3 is expansively spread outward along the concave portion 25 and is capable of easily climbing over the head portion 24.

In the seal cap 3 of this embodiment, the hollow part 30 has a piercing needle mounting part-introducing portion 38 which is formed in a range from the open proximal end part 32 of the seal cap 3 to the proximal end of the piercing needle fixing part-accommodating part 35 (projected part 36) and is extended in substantially the same inner diameter. The piercing needle mounting part-introducing portion 38 has an inner diameter a little larger than a maximum inner diameter of the piercing needle fixing part-accommodating part 35 and a little larger than the outer diameter of the head portion 24 of the piercing needle fixing part 22 of the outer cylinder 2. Therefore, in mounting the seal cap 3 on the piercing needle fixing part 22 of the outer cylinder 2, the piercing needle mounting part-introducing portion 38 functions as a portion for introducing the piercing needle mounting part 2 into the seal cap.

The piercing needle mounting part-introducing portion 38 has an annular erect surface 39 erect toward the open proximal end part 32 at a boundary between the piercing needle mounting part-introducing portion and the proximal end of the piercing needle fixing part-accommodating part 35 (projected part 36). Thus, when the distal end of the outer cylinder 2 is inserted into the piercing needle mounting part-introducing portion 38 of the seal cap 3, the piercing needle fixing part 22 of the outer cylinder 2 enters the piercing needle mounting part-introducing portion 38. Thereafter as shown in Fig. 10, an annular distal end surface of the head portion 24 of the piercing needle fixing part 22 contacts the annular erect surface 39. In this state, the piercing needle 6 becomes substantially parallel with the central axis of the seal cap 3 and has a state in which the piercing needle enters the small-diameter distal end portion 34a of the piercing needle accommodating part 34.

A gripping flange 37 is formed at the proximal end portion of the seal cap 3 by projecting the flange outward and annularly. An annular concave portion 71 is formed on the flange 37. The distal end position of the flange 37 is distal from the annular erect surface 39 of the hollow part 30 and in the vicinity of the apex 36a of the projected part 36 (in the case of the flange shown in Fig. 6, the distal end position of the flange is located at a position a little proximal from the apex 36a).

The method for producing the seal cap of the present invention is described below.

The method for producing the seal cap of the present invention produces the seal cap to be mounted on the needle-attached outer cylinder having the outer cylinder body having the piercing needle fixing part at its distal end and the piercing needle which has the piercing needle tip at its distal end and whose proximal end portion is inserted into the piercing needle fixing part and fixed thereto.

The method for producing the seal cap of the present invention includes the step of preparing the seal cap which has the cylindrical hollow part accommodating the piercing needle fixing part of the needle-attached outer cylinder therein and the pierceable part into which the piercing needle tip of the piercing needle inserted into the hollow part is pierceable and which is formed of the rubber composition containing the mixture of butyl rubber and diene-based rubber as its base polymer; and an ozone contact step of exposing the seal cap to a high concentration ozone environment.

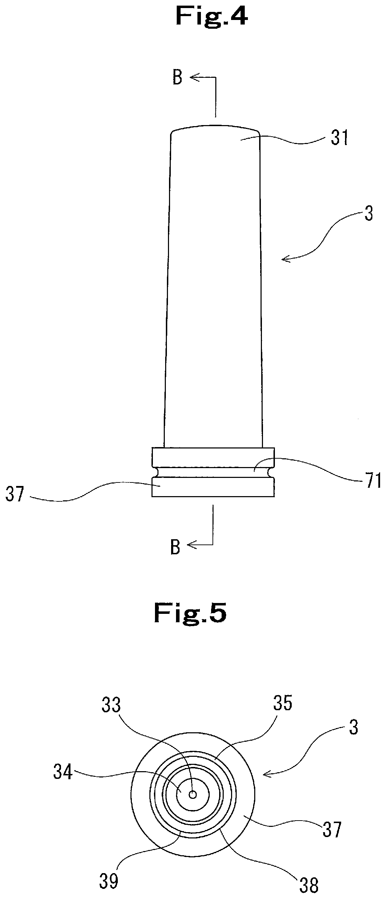

At the step of preparing the seal cap, the seal cap body having the form, as shown in Figs. 4 through 6, which has the cylindrical hollow part accommodating the piercing needle fixing part of the needle-attached outer cylinder therein and the pierceable part into which the piercing needle tip of the piercing needle 6 inserted into the hollow part is pierceable is prepared.

At the step of preparing the seal cap body, a seal cap body produced by using the above-described rubber composition as the material for the seal cap is prepared. It is possible to prepare the seal cap body produced by using the rubber composition and having the sticking restraining film formed on the inner surface of the piercing needle mounting part-accommodating part of the plastic part of the seal cap.

Thereafter the ozone contact step of exposing the seal cap to the high concentration ozone environment is performed.

The high concentration ozone environment includes a static electricity removal environment adopting a corona discharge method, the inside of an ozone sterilizer, the inside a clean room where ozone is sterilization, a space (for example, hospital) in which an air cleaner generating ozone is used. When the seal cap and the outer cylinder on which the seal cap has been mounted are charged with static electricity, the seal cap and the outer cylinder may adsorb dust and the like during production processes. Therefore, the production processes are performed in the static electricity removal environment from which the static electricity is removed. The static electricity removal environment can be formed by introducing ions generated by using an ionizer adopting the corona discharge method and pure air into the production processes. At a corona discharge time, ions and ozone are generated. As a result, the outer cylinder on which the seal cap has been mounted contacts the ozone. But because the seal cap of the present invention mounted on the outer cylinder has an ozone-resistant outer surface covering layer as described above, the seal cap is restrained from being deteriorated by the ozone. Therefore, it hardly occurs that the seal cap deteriorates owing to the execution of the ozone contact process. The method to be used to form the static electricity removal environment is not limited to the corona discharge method (any of voltage application method and self-discharge method can be used), but it is possible to use an AC discharge method, a DC discharge method or a pulse discharge method.

### EXAMPLES

As a material for forming a seal cap, butyl rubber (HT-1066 produced by Japan Butyl Co., Ltd.), butadiene rubber (BR01 produced by JSR Co., Ltd.), filler (silica VN3 produced by Tosoh Silica Corporation and talc K-1 produced by Nippon Talc Co., Ltd.), and stearic acid amide which is a release agent were prepared. These components used at mixing ratios shown in table 1, a crosslinking agent, and a catalyst were kneaded by using a closed type kneading machine to obtain unvulcanized rubber compounds. The unvulcanized rubber compounds were pressurized and heated to crosslink them by using a predetermined molding die. In this manner, seal caps having a configuration as shown in Fig. 2 were produced.

**Table 1**

| Material (parts by weight) | | Example 1 | Example 2 | Example 3 |
|---|---|---|---|---|
| composition | Rubber component | 70 | 60 | 70 |
| | Filler | 30 | 40 | 30 |
| | Release agent 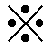 | 2 | 2 | 2 |
| Rubber component | Butyl rubber | 50 | 60 | 70 |
| | Butadiene rubber | 50 | 40 | 30 |

| | | | | |
|---|---|---|---|---|
| 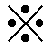 : ratio (%) when rubber component is converted to 100% | | | | |

**Table 2**

| Material (parts by weight) | | Comparison example 1 | Comparison example 2 |
|---|---|---|---|
| Composition | Rubber component | 70 | 60 |
| | Filler | 30 | 40 |
| | Release agent 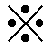 | 2 | 2 |
| Rubber component | Butyl rubber | 100 | 0 |
| | Butadiene rubber | 0 | 100 |

| | | | |
|---|---|---|---|
| 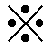 : ratio (%) when rubber component is converted to 100% | | | |

By using a 29-gauge piercing needle made of stainless steel and cyclic olefin polymer (COP) which is a cyclic olefin homopolymer as the resin for forming the outer cylinder, needle-attached outer cylinders which have a form as shown in Figs. 7 through 9 and on which the above-described seal caps can be mounted were produced by insert molding. After applying a lubricant (silicone oil) to the needle tips of the piercing needles, the seal caps of the examples 1 through 3 and the comparison examples 1 and 2 were mounted on the needle-attached outer cylinders respectively. In this way, syringe assemblies in each of which the needle tip of the piercing needle was pierced into the seal cap were produced. 150 syringe assemblies of each of the examples 1 through 3 and the comparison examples 1 and 2 were produced by subjecting them to high-pressure steam sterilization.

### (Experiment 1: Fixing Checking Test)

A fixing checking test was conducted on 100 syringe assemblies of each of the examples 1 through 3 and the comparison examples 1 and 2 produced as described above and sterilized. In the test, the seal cap was removed from each syringe assembly to check whether the material forming the seal cap stuck to the vicinity of needle tip of the piercing needle and counted the number of the piercing needles to which the material for the seal cap stuck. Table 3 shows the results. In the fixing test column of table 3, the mark of "∘" shows that the material forming the seal cap did not stick to the needle tip (needle shielding rubber piece whose area was larger than 0.03mm² stuck to none of the needle tips) and the mark of "×" shows that the material forming the seal cap stuck to the needle tip (needle shielding rubber piece whose area was larger than 0.03mm² stuck to one or more needle tips). As a result of the conduct of the experiment, it could be confirmed that in the syringe assemblies using the seal caps of the examples of the present invention, the material forming the seal cap did not stick to the sterilized piercing needle.

### (Measurement of Water Vapor Permeability)

The unvulcanized rubber compound of each of the examples 1 through 3 and the comparison examples 1 and 2 was vulcanized and molded into a sheet to measure the water vapor permeability of the sheet. To measure the water vapor permeability, a water vapor test was conducted at 125 degrees C in accordance with "Plastics - Film - sheeting - Determination of water vapour transmission rate Instrumental method" described in JIS K7129 (revised on 2008/03/20). The test results were as shown in table 3. As a result of the conduct of the experiment, it could be confirmed that the material forming the seal cap of the examples had satisfactory water vapor permeability.

### (Experiment 3: Sterility Test)

By using the seal cap and needle-attached outer cylinders of each of the examples 1 through 3 and the comparison examples 1 and 2 produced as described above and sterilized, BI bacteria (2.7 × 10⁶CFU/ml) was filled in each seal cap, 20 syringe assemblies in each of which the seal cap was mounted on the outer cylinder were prepared. Because the internal pressure inside the seal cap rises when the seal cap is mounted on the outer cylinder, the neighborhood of an open portion of the seal cap was deformed to form a gap between the outer cylinder and the seal cap so that the internal pressure was released. After the seal cap was mounted on the outer cylinder, the syringe assemblies were subjected to autoclave treatment (123 degrees C, 80 minutes). After the syringe assemblies were sterilized, culture medium treatment was performed for seven days. The results were as shown in table 3. In the sterility column of table 3, the mark of "o" shows that the BI bacteria could be sterilized (all bacteria were killed) and the mark of "×" shows that the BI bacteria could not be sterilized (none of bacteria was killed). It could be confirmed that the seal caps of the examples had autoclave sterilization performance.

### (Experiment 4: Ozone Resistance Test)

In accordance with "Rubber, vulcanized or thermoplastic - Determination of ozone resistance" described in JIS K 6249 (revised on 2004/03/20), a test was conducted on 10 syringe assemblies of each of the examples 1 through 3 and the comparison examples 1 and 2 produced as described above and sterilized. After the syringe assemblies were exposed to a high concentration ozone environment, they were checked whether they were resistant to ozone. The results were as shown in table 3. The mark of "o" in table 3 shows that the seal cap was resistant to ozone (seal cap was not deteriorated by ozone in its appearance) and the mark of "×" shows that the seal cap was not resistant to ozone (seal cap was deteriorated by ozone in its appearance). It could be confirmed that the seal caps of the examples were resistant to ozone.

### (Experiment 5: Piercing Resistance Test)

A piercing resistance test was conducted on 20 syringe assemblies of each of the examples 1 through 3 and the comparison examples 1 and 2 produced as described above and sterilized. After the seal cap was removed from the outer cylinder, the piercing resistance of each needle tip to silicone rubber (t=0.5mm) was measured by using an autograph. The results were as shown in table 3. The needle tip having a low piercing resistance is preferable.

It could be confirmed that the needle tips pierced into the seal caps of the examples respectively had a sufficiently high piercing property.

**Table 3**

| | Fixing test | Water vapor permeability | Sterility | Ozone resistance | Piercing resistance |
|---|---|---|---|---|---|
| Example 1 | ○ | 500 | ○ | ○ | ○/0.15N |
| Example 2 | ○ | 480 | ○ | ○ | ○/0.15N |
| Example 3 | ○ | 470 | ○ | ○ | ○/0.14N |
| Comparison example 1 | ○ | 200 | × | ○ | ○/0.15N |
| Comparison example 2 | × | 680 | ○ | × | ○/0.14N |

In the syringe assembly, the seal cap is formed of the rubber composition containing the mixture of butyl rubber and diene-based rubber as its base polymer. Therefore, when the seal cap is mounted on the outer cylinder, it hardly occurs that the outer surface of a portion of the seal cap pressed by its inside deteriorates or crackles owing to the contact between the outer surface thereof and ozone. In the syringe assembly of the present invention, although the inner surface of the piercing needle mounting part-accommodating part of the seal cap and the outer surface of the piercing needle mounting part of the outer cylinder are in close contact with each other, the seal cap has excellent autoclave sterilization performance because the seal cap has satisfactory water vapor permeability. When the seal cap into which the piercing needle has been pierced is removed from the outer cylinder with the lapse of a certain period of time, it does not occur that the material forming the seal cap is left on the outer surface of the piercing needle.

## Claims

1. A syringe assembly (10) comprising:
an outer cylinder (2) having an outer cylinder body part (21), a piercing needle mounting part (22) provided at a distal end portion of said outer cylinder body part (21),
a piercing needle (6) which has a piercing needle tip (61) at a distal end thereof and whose proximal end portion is fixed to said piercing needle mounting part (22), and a seal cap (3) mounted on said outer cylinder (2),
wherein said seal cap (3) comprises a closed distal end part (31), an open proximal end part (32), a hollow part (30) including a piercing needle mounting part-accommodating part positioned at a distal side from said open proximal end part (32) and accommodating a distal end portion of said piercing needle mounting part (22) and a piercing needle accommodating part continuous with a distal end of said piercing needle mounting part-accommodating part and accommodating said piercing needle (6), and a pierceable part (33) into which said piercing needle tip (61) of said piercing needle accommodated inside said piercing needle accommodating part is pierceable, wherein said seal cap (10) is mounted on said outer cylinder (21), and wherein said piercing needle tip (61) is in a state of being pierced into said pierceable part (33) of said seal cap (3),
**characterized in that**
said seal cap (3) is formed of a rubber composition containing a mixture of butyl rubber and diene-based rubber as a base polymer thereof, the ratio between said butyl rubber and said diene-based rubber of said rubber composition being set from 7:3 to 4:6, and
said syringe assembly (10) is subjected to high-pressure steam sterilization.

2. A syringe assembly (10) according to claim 1, wherein said diene-based rubber contains butadiene rubber as a main component thereof.

3. A syringe assembly (10) according to claim 1, wherein said diene-based rubber is butadiene rubber.

4. A syringe assembly (10) according to any one of claims 1 through 3, wherein said rubber composition contains a filler.

5. A syringe assembly (10) according to claim 4, wherein said filler is an inorganic filler.

6. A syringe assembly (10) according to claim 4 or 5, wherein the ratio between said base polymer of said rubber composition and the filler is set from 5:5 to 8:2.

7. A syringe assembly (10) according to any one of claims 1 through 6, wherein said butyl rubber of said rubber composition and said diene-based rubber thereof are crosslinked.

8. A syringe assembly (10) according to any one of claims 1 through 7, wherein said piercing needle (6) is a hollow needle having said piercing needle tip (61) at a distal end thereof and communicates with an inner space (20) of said outer cylinder body part (21) at a proximal end portion thereof.

9. A syringe assembly (10) according to claim 8, wherein an inner surface of said piercing needle mounting part-accommodating part of said seal cap (3) and an outer surface of said piercing needle mounting part of said outer cylinder (2) are in close contact with each other.

10. A syringe assembly (10) according to claim 1, wherein said seal cap (3) contains a release agent, and said release agent is high fatty acid amide, high fatty acid ester, high fatty acid amine, ester of aliphatic alcohol or paraffin.

11. A prefilled syringe comprising a syringe assembly (10) according to any one of claims 1 through 8, a gasket (4) which is accommodated inside said outer cylinder (2) and liquid-tightly slidable inside said outer cylinder (2), and a medical agent (8) filled inside a space formed of said outer cylinder (2) and said gasket (4).

12. A syringe assembly packaging body accommodating a plurality of syringe assemblies (10) according to any one of claims 1 through 10,
said packaging body comprising a container body whose upper surface is open and which has shape retainability, an outer cylinder (2) holding member capable of holding a plurality of said syringe assemblies (10), a plurality of said syringe assemblies (10) held by said outer cylinder holding member, and a sheet-shaped lid member which airtightly seals said open upper surface of said container body and is peelable therefrom, and an air-permeable part, having bacteria impermeability and sterilizing gas permeability, which is provided on said container body or on said sheet-shaped lid member, and said packaging body has subjected to high-pressure steam sterilization.

## Patentansprüche

1. Spritzenanordnung (10), umfassend:
einen äußeren Zylinder (2), der einen äußeren Zylinderkörperteil (21) aufweist, einen Einstichnadel-Befestigungsteil (22), der an einem distalen Endabschnitt des äußeren Zylinderkörperteils (21) vorgesehen ist,
eine Einstichnadel (6), die eine Einstichnadelspitze (61) an ihrem distalen Ende davon aufweist und deren proximaler Endabschnitt an dem Einstichnadel-Befestigungsteil (22) befestigt ist, und eine Verschlusskappe (3), die auf dem äußeren Zylinder (2) angebracht ist,
wobei die Verschlusskappe (3) ein geschlossenes distales Endteil (31), ein offenes proximales Endteil (32), ein hohles Teil (30), das ein Aufnahmeteil des Einstichnadel-Befestigungsteils, welches an einer distalen Seite von dem offenen proximalen Endteil (32) positioniert ist und einen distalen Endabschnitt des Einstichnadel-Befestigungsteils (22) aufnimmt, und ein Einstichnadel-Aufnahmeteil aufweist, das mit einem distalen Ende von dem Aufnahmeteil des Einstichnadel-Befestigungsteils zusammenhängt und die Einstichnadel (6) aufnimmt, und einen einstechbaren Teil (33) umfasst, in den die Einstichnadelspitze (61) der Einstichnadel, welche in dem Einstichnadel-Aufnahmeteil untergebracht ist, einstechbar ist, wobei die Verschlusskappe (10) an dem äußeren-Zylinder (21) angebracht ist und wobei die Einstichnadelspitze (61) in einem Zustand ist, in welchem sie in den einstechbaren Teil (33) der Verschlusskappe (3) eingestochen wird,
**dadurch gekennzeichnet, dass**
die Verschlusskappe (3) aus einer Kautschukzusammensetzung gebildet ist, die eine Mischung aus Butylkautschuk und Kautschuk auf Dien-Basis als ein Basispolymer davon enthält, wobei das Verhältnis zwischen dem Butylkautschuk und dem Kautschuk auf Dien-Basis der Kautschukzusammensetzung auf 7:3 bis 4:6 eingestellt ist, und
die Spritzenanordnung (10) einer HochdruckDampfsterilisation unterzogen wird.

2. Spritzenanordnung (10) nach Anspruch 1, wobei der Kautschuk auf Dienbasis Butadienkautschuk als eine Hauptkomponente davon enthält.

3. Spritzenanordnung (10) nach Anspruch 1, wobei der Kautschuk auf Dienbasis Butadienkautschuk ist.

4. Spritze (10) nach einem der Ansprüche 1 bis 3, wobei die Kautschukzusammensetzung einen Füllstoff enthält.

5. Spritzenanordnung (10) nach Anspruch 4, wobei der Füllstoff ein anorganischer Füllstoff ist.

6. Spritzenanordnung (10) nach Anspruch 4 oder 5, wobei das Verhältnis zwischen dem Basispolymer der Kautschukzusammensetzung und dem Füllstoff auf 5:5 bis 8:2 eingestellt ist.

7. Spritzenanordnung (10) nach einem der Ansprüche 1 bis 6, wobei der Butylkautschuk der Kautschukzusammensetzung und der Kautschuk auf Dienbasis davon vernetzt sind.

8. Spritzenanordnung (10) nach einem der Ansprüche 1 bis 7, wobei die Einstichnadel (6) eine Hohlnadel ist, welche die Einstichnadelspitze (61) an einem distalen Ende davon aufweist und mit einem Innenraum (20) des äußeren Zylinderkörperteils (21) an einem proximalen Endabschnitt davon in Verbindung steht.

9. Spritzenanordnung (10) nach Anspruch 8, wobei eine Innenfläche von dem Aufnahmeteil des Einstichnadel-Befestigungsteils der Verschlusskappe (3) und eine Außenfläche des Einstichnadel-Befestigungsteils des äußeren Zylinders (2) in engem Kontakt miteinander stehen.

10. Spritzenanordnung (10) nach Anspruch 1, wobei die Verschlusskappe (3) ein Trennmittel enthält und das Trennmittel ein Amid mit hohem Fettsäuregehalt, ein Ester mit hohem Fettsäuregehalt, ein Amin mit hohem Fettsäuregehalt, ein Ester eines aliphatischen Alkohols oder Paraffin ist.

11. Vorgefüllte Spritze, umfassend eine Spritzenanordnung (10) nach einem der Ansprüche 1 bis 8, eine Dichtung (4), die im Inneren des äußeren Zylinders (2) untergebracht ist und die flüssigkeitsdicht im Inneren des äußeren Zylinders (2) verschiebbar ist, und ein medizinisches Mittel (8), das in das Innere eines Raumes gefüllt ist, der von dem äußeren Zylinder (2) und der Dichtung (4) gebildet wird.

12. Verpackungskörper für Spritzenanordnung, der eine Vielzahl von Spritzenanordnungen (10) nach einem der Ansprüche 1 bis 10 aufnimmt,
wobei der Verpackungskörper einen Behälterkörper, dessen Oberseite offen ist und der eine Formbeständigkeit aufweist, ein Halteelement für den äußeren Zylinder (2), das in der Lage ist, eine Vielzahl der Spritzenanordnungen (10) für Spritzen zu halten, eine Vielzahl der Spritzenanordnungen (10), die von dem Halteelement für den äußeren Zylinder gehalten werden, und ein blattförmiges Deckelelement (103), das die offene Oberseite des Behälterkörpers luftdicht verschließt und von diesem abziehbar ist, umfasst und einen luftdurchlässigen Teil, der eine Bakterienundurchlässigkeit und Sterilisationsgasdurchlässigkeit aufweist, der an dem Behälterkörper oder an dem blattförmigen Deckelelement vorgesehen ist, und der Verpackungskörper einer Hochdruckdampfsterilisation unterzogen wurde.

## Revendications

1. Ensemble de seringue (10) comprenant :
un cylindre extérieur (2) présentant une partie de corps de cylindre extérieur (21), une partie de montage d'aiguille de perçage (22) prévue au niveau d'une partie d'extrémité distale de ladite partie de corps de cylindre extérieur (21),
une aiguille de perçage (6) qui présente une pointe d'aiguille de perçage (61) au niveau d'une extrémité distale de celle-ci et dont la partie d'extrémité proximale est fixée à ladite partie de montage d'aiguille de perçage (22), et un capuchon d'étanchéité (3) monté sur ledit cylindre extérieur (2),
dans lequel ledit capuchon d'étanchéité (3) comprend une partie d'extrémité distale fermée (31), une partie d'extrémité proximale ouverte (32), une partie creuse (30) comportant une partie de réception de partie de montage d'aiguille de perçage positionnée au niveau d'un côté distal à partir de ladite partie d'extrémité proximale ouverte (32) et recevant une partie d'extrémité distale de ladite partie de montage d'aiguille de perçage (22) et une partie de réception d'aiguille de perçage continue avec une extrémité distale de ladite partie de réception de partie de montage d'aiguille de perçage et recevant ladite aiguille de perçage (6), et une partie pouvant être percée (33) dans laquelle ladite pointe d'aiguille de perçage (61) de ladite aiguille de perçage reçue à l'intérieur de ladite partie de réception d'aiguille de perçage peut être percée, dans lequel ledit capuchon d'étanchéité (10) est monté sur ledit cylindre extérieur (21), et dans lequel ladite pointe d'aiguille de perçage (61) est dans un état dans lequel elle est percée dans ladite partie pouvant être percée (33) dudit capuchon d'étanchéité (3),
**caractérisé en ce que**
ledit capuchon d'étanchéité (3) est formé d'une composition de caoutchouc contenant un mélange de butylcaoutchouc et caoutchouc à base de diène comme polymère de base de celui-ci, le rapport entre ledit butylcaoutchouc et ledit caoutchouc à base de diène de ladite composition de caoutchouc étant réglé de 7:3 à 4:6, et
ledit ensemble de seringue (10) est soumis à une stérilisation par vapeur à haute pression.

2. Ensemble de seringue (10) selon la revendication 1, dans lequel ledit caoutchouc à base de diène contient du caoutchouc butadiène comme composant principal de celui-ci.

3. Ensemble de seringue (10) selon la revendication 1, dans lequel ledit caoutchouc à base de diène est du caoutchouc butadiène.

4. Ensemble de seringue (10) selon l'une quelconque des revendications 1 à 3, dans lequel ladite composition de caoutchouc contient une charge.

5. Ensemble de seringue (10) selon la revendication 4, dans lequel ladite charge est une charge inorganique.

6. Ensemble de seringue (10) selon la revendication 4 ou 5, dans lequel le rapport entre ledit polymère de base de ladite composition de caoutchouc et la charge est réglé de 5:5 à 8:2.

7. Ensemble de seringue (10) selon l'une quelconque des revendications 1 à 6, dans lequel ledit butylcaoutchouc de ladite composition de caoutchouc et ledit caoutchouc à base de diène de celui-ci sont réticulés.

8. Ensemble de seringue (10) selon l'une quelconque des revendications 1 à 7, dans lequel ladite aiguille de perçage (6) est une aiguille creuse présentant ladite pointe d'aiguille de perçage (61) au niveau d'une extrémité distale de celle-ci et communique avec un espace intérieur (20) de ladite partie de corps de cylindre extérieur (21) au niveau d'une partie d'extrémité proximale de celle-ci.

9. Ensemble de seringue (10) selon la revendication 8, dans lequel une surface intérieure de ladite partie de réception de partie de montage d'aiguille de perçage dudit capuchon d'étanchéité (3) et une surface extérieure de ladite partie de montage d'aiguille de perçage dudit cylindre extérieur (2) sont en contact étroit l'une avec l'autre.

10. Ensemble de seringue (10) selon la revendication 1, dans lequel ledit capuchon d'étanchéité (3) contient un agent de démoulage, et ledit agent de démoulage est un amide d'acide gras élevé, un ester d'acide gras élevé, une amine d'acide gras élevé, un ester d'alcool aliphatique ou une paraffine.

11. Seringue préremplie comprenant un ensemble de seringue (10) selon l'une quelconque des revendications 1 à 8, un joint d'étanchéité (4) qui est reçu à l'intérieur dudit cylindre extérieur (2) et peut coulisser de manière étanche au liquide à l'intérieur dudit cylindre extérieur (2), et un agent médical (8) versé à l'intérieur d'un espace formé dudit cylindre extérieur (2) et dudit joint d'étanchéité (4).

12. Corps d'emballage d'ensemble de seringue recevant une pluralité d'ensembles de seringue (10) selon l'une quelconque des revendications 1 à 10,
ledit corps d'emballage comprenant un corps de contenant dont la surface supérieure est ouverte et qui présente une capacité de conservation de forme, un élément de retenue de cylindre extérieur (2) apte à retenir une pluralité desdits ensembles de seringue (10), une pluralité desdits ensembles de seringue (10) retenus par ledit élément de retenue de cylindre extérieur, et un élément de couvercle en forme de feuille qui étanchéifie de manière étanche à l'air ladite surface supérieure ouverte dudit corps de contenant et peut en être décollée, et une partie perméable à l'air, présentant une imperméabilité aux bactéries et une perméabilité aux gaz de stérilisation, qui est prévue sur ledit corps de contenant ou sur ledit élément de couvercle en forme de feuille, et ledit corps d'emballage se voit soumis à une stérilisation par vapeur à haute pression.
